# EUROPEAN PATENT APPLICATION

(11) **EP 1 444 977 A1**
(43) Date of publication of application: **11.08.2004**
(21) Application number: 04002041.4
(22) Date of filing: 30.01.2004
(51) Int. Cl.: A61K 9/70, A61K 47/12, A61K 47/22

(54) **Transdermal therapeutic delivery system with a butenolide**

(30) Priority: 07.02.2003 DE 10305137
(71) Applicant: Novosis AG, 83714 Miesbach (DE)
(72) Inventor: Schenk, Dirk, 83623 Dietramszell (DE); Meyer, Elisabeth, 83714 Miesbach (DE); WöSS Angelika, 83714 Miesbach (DE); McLeod Sarah, 81739 München (DE)

(57) **Abstract**

The invention concerns a transdermal therapeutic delivery system (TTDS) with butenolide.

## Description

The stability of drug compounds in transdermal therapeutic systems is a prerequisite of highest priority. Oxidation of the drug is therefore often prevented by addition of antioxidants, e.g. vitamin C, vitamin E, butylhydroxytoluene, Controx KS, hydroquinone etc.

A prominent impurity in transdermal formulations results from oxidation processes of drugs during storage. As a main oxidation product, the corresponding N-oxide is often observed. N-oxide derivatives of a number of alkaloids can considerably affect the activity of biological macromolecules (haemoglobin, cytochromes, cytochrome P-450, superoxide dismutase, catalase, and others), for instance, modulate the electron transport function of cytochromes. Taking into account the possible biological action of these compounds, they may be considered in particular, as respiratory poisons.

WO 96/06 602 describes adhesive matrices containing 5-aminolevulinic acid, pharmaceutically acceptable salts thereof and products, which are considered pharmaceutical equivalents, further optionally a stabilising amount of an organic weak proton donor of a saccharide and other components, such as penetration enhancer and other substances known for use in transdermal formulations. Further, levulinic acid has been used in transdermal therapeutic systems as co-component of active agents as can be drawn from the following table:

| | active agent | levulinic acid [MW 116]: active agent | |
|---|---|---|---|
| | | weight | mol |
| DE 197 38 855 | buprenorphin-base [MW 468] | 1:1 | 4:1 |
| DE 198 34 005 | morphin-alkaloid | | 1:1 |
| DE 199 06 977 | desoxy peganin | 1:1 | 1.5:1 |
| [1] and WO/19 975 | buprenorphin-base | 1:1 | 4:1 |

The invention starts from the surprising finding that levulinic acid exerts antioxidative effects of high potency. The antioxidative mechanism of levulinic acid might be explained by the fact that it might undergo formation to unsaturated γ-lactone [2], representing itself a target for oxidation processes.

The problem from which the invention starts is solved by a transdermal therapeutic delivery system (TTDS) comprising one, two or more drugs and a butenolide. As regards the nomenclature of butenolides reference is made to Chemical Reviews, 64 (1964) 353-388 [3].

According to the invention the butenolide may be α-angelica lactone (4-hydroxy-3-pentenoic acid γ-lactone) or β-angelica lactone (4-hydroxy-2-pentenoic acid γ-lactone).

Further, the problem from which the invention starts is solved by a transdermal therapeutic delivery system (TTDS) comprising one, two or more drugs and a precursor of a butenolide, wherein the following drugs are excluded as drug: buprenorphin-base, desoxy peganin and an addition salt of levulinic acid and a morphin alkaloid of the following formula 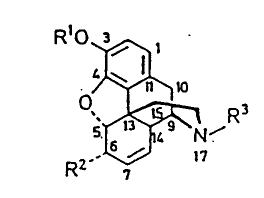 wherein
R¹ is H, C₁₋₆-alkyl group or COCH₃,
R² is H, OH, OCOCH₃ , =O or =CH₂ and
R³ is CH₃, cyclopropyl, cyclobutyl or allyl, and wherein the C7/C8 linkage can be saturated or at N₁₇ a nitroxyl group can be provided.

Further, the problem from which the invention starts is solved by a transdermal therapeutic delivery system (TTDS) comprising one, two or more drugs and a precurser of a butenolide wherein the molar ratio of drug(s): precurser is of from 1:4.1 to 1:15 and especially 1:4.5 to 1:15 and more especially 1:4.5 to 1:10.

According to the invention the precurser of the butenolide can be levulinic acid or 5-amino-levulinic acid.

Further, according to the invention the drug(s) may comprise an N-atom susceptible to oxidation to an N-oxide.

Further, according to the invention the drug(s) may be an alkaloid, especially an alkaloid selected from the group consisting of opioid and/or ergot alkaloids.

Further, according to the invention the drug(s) may be a quinoline and/or a pyridine derivative.

Further, according to the invention the system may be of the matrix-type or of the reservoir-type.

Further, according to the invention the system may comprise a backing layer, a removable protecting layer and a matrix or a reservoir in between the backing layer and the protecting layer.

Further, according to the invention the system may be of the reservoir type and comprise a membrane.

Further, according to the invention the system may comprise a butenolide or a precursor thereof as antioxidant.

Further, according to the invention the amount of the butenolide may be adjusted to the intended time of storage. Thus, according to the invention
- for use of the system in a temperate climate the molar ratio of drug(s): precursor or butenolide may be of from 1:4.1 to 1 to 1:5 and
- for use of the system in a subtropical or tropical climate the molar ratio of drug(s): precursor or butenolide may be of from 1:4.5 to 1:15.

### Examples 1 to 2 and Comparative Examples 1 to 5

We have employed levulinic acid as antioxidative substance in transdermal therapeutic systems to prevent oxidation of opioids and ergot alkaloids. The first (opioid) system (Example 1) describes the use of hydromorphone, the 6-oxo derivative of morphine, which selectively binds the µ-opioid receptors. The composition of the hydromorphone formulation was: 1.3% hydromorphone base, 10% levulinic acid, 5% dexpanthenol, 2% Klucel, ad 100% with ethanol/aqua purificata mixture 1:1. In the second system (Example 2), Lisurid, an ergot alkaloid has been employed. Lisurid is a dopamine agonist exerting antiparkinsonian effects by acting directly on dopamine receptors and mimicking the endogenous neurotransmitter. The formulation used contained 1% Lisurid base, 10% Copherol, 5% levulinic acid, 2% Klucel, 82% 2-propanol. The solutions were stored for 7 days at 40°C. Analytics were performed by means of ion pair reversed phase HPL-Chromatography. The results demonstrate highest efficacy of levulinic acid in comparison to conventional antioxidants (Comparative Examples 1 to 5). Whereas substances as vitamin C, vitamin E, butylhydroxytoluene, Controx KS and hydroquinone led to N-oxide contents of the active of up to 41% peak area, N-oxide content of the formulation employing levulinic acid was below 0.06%.

### References

[1] Hille, T.: Transdermal resorption of active substances from supercooled masses. LTS Lohmann Therapie-Systeme GmbH. WO 96/19975
[2] Hans Beyer: Lehrbuch der organischen Chemie. Ed. Wolfgang Walter. 20. Auflage. S.Hirzel Verlag Stuttgart, 1984. p.281 f
[3] Rao, Y.S.: Chemistry of Butenolides. Chem. Reviews, 1964, 64: 353

## Claims

1. Transdermal therapeutic delivery system (TTDS) comprising one, two or more drugs and a butenolide.

2. System according to claim 1 wherein the butenolide is a α-angelica lactone (4-hydroxy-3-pentenoic acid gamma-lactone) or β-angelica lactone (4-hydroxy-2-pentenoic acid gamma-lactone).

3. Transdermal therapeutic delivery system (TTDS) comprising one, two or more drugs and a precursor of a butenolide, wherein the following drugs are excluded as drug:
buprenorphin-base, desoxy peganin and an addition salt of levulinic acid and a morphin alkaloid of the following formula 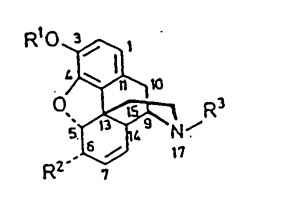
wherein
R¹ is H, C₁₋₆-alkyl group or COCH₃,
R² is H, OH, OCOCH₃, =O or =CH₂ and
R³ is CH₃, cyclopropyl, cyclobutyl or allyl, and wherein the C7/C8 linkage can be saturated or at N₁₇ a nitroxyl group can be provided.

4. Transdermal therapeutic delivery system (TTDS) comprising one, two or more drugs and a precursor of a butenolide wherein the mole ratio of drug(s): precursor is of from 1:4.1 to 1:15 and especially 1:4.5 to 1:10.

5. System according to claim 3 or 4 wherein the precursor of the butenolide is levulinic acid or 5-amino-levulinic acid.

6. System according to claim 1, 2, 3, 4, 5 or a combination thereof wherein the drug(s) comprises (comprise) an N-atom susceptible to oxidation to an N-oxide.

7. System according to claim 1, 2, 3, 4, 5, 6 or a combination thereof wherein the drug(s) is (are) an alkaloid, especially an alkaloid selected from the group consisting of opioids and/or ergot alkaloids.

8. System according to claim 1, 2, 3, 4, 5, 6, 7 or a combination thereof wherein the drug(s) is (are) a quinoline and/or a pyridine derivative.

9. System according to claim 1, 2, 3, 4, 5, 6, 7, 8 or a combination thereof wherein the system is of the matrix-type or of the reservoir-type.

10. System according to claim 9 wherein the system comprises a backing layer, a removable protecting layer and a matrix or a reservoir in between the backing layer and the protecting layer.

11. System according to claim 9 and/or 10 wherein the system is of the reservoir-type and comprises a membrane.

12. System according to claim 1, 2, 3, 4, 5, 6, 7, 8 ,9, 10, 11 or a combination thereof comprising a butenolide or a precursor thereof as antioxidant.

13. System according to claim 12 wherein the amount of the butenolide is adjusted to the intended time of storage.

14. System according to claim 12 and/or 13 wherein
- for use of the system in a temperate climate the molar ratio of drug(s): precursor or butenolide is of from 1:4.1 to 1:5 and
- for use of the system in a subtropical or tropical climate the molar ratio of drug(s): precursor or butenolide is of from 1:4.1 to 1:15.
